# EUROPÄISCHE PATENTSCHRIFT

(11) **EP 2 617 110 B1**
(45) Veröffentlichungstag und Bekanntmachung des Hinweises auf die Patenterteilung: **08.03.2017**
(21) Anmeldenummer: 11766905.1
(22) Anmeldetag: 12.09.2011
(51) Int. Cl.: H01S 5/22

(54) **LASERDIODE MIT HOHER EFFIZIENZ**
LASER DIODE WITH HIGH EFFICIENCY
DIODE LASER PRÉSENTANT UNE EFFICACITÉ ÉLEVÉE

(30) Priorität: 14.09.2010 DE 102010040767
(43) Veröffentlichungstag der Anmeldung: 24.07.2013
(73) Patentinhaber: Forschungsverbund Berlin E.V., 12489 Berlin (DE)
(72) Erfinder: CRUMP, Paul, 10245 Berlin (DE); ERBERT, Götz, 02708 Löbau (DE); WENZEL, Hans, 12355 Berlin (DE)
(74) Vertreter: Gulde & Partner
(86) Internationale Anmeldenummer: PCT/EP2011/065751
(87) Internationale Veröffentlichungsnummer: WO 2012/034972

(56) Entgegenhaltungen:
- EP-A1- 1 460 741
- WO-A1-01/57974
- US-A1- 2003 179 794
- US-A1- 2004 066 818
- US-A1- 2008 036 044
- US-A1- 2008 117 945
- US-B1- 6 731 663
- KIMIO SHIGIHARA ET AL: "High-Power 980-nm Ridge Waveguide Laser Diodes Including an Asymmetrically Expanded Optical Field Normal to the Active Layer", IEEE JOURNAL OF QUANTUM ELECTRONICS, IEEE SERVICE CENTER, PISCATAWAY, NJ, USA, Bd. 38, Nr. 8, 1. August 2002 (2002-08-01) , XP011065253, ISSN: 0018-9197

## Beschreibung

Die vorliegende Erfindung betrifft eine Laserdiode mit hoher Effizienz und hoher Augensicherheit.

In vielen Anwendungsgebieten, beispielsweise der Kosmetik (Haarentfernung), der Beleuchtung oder der Datenübertragung werden effiziente Lichtquellen nachgefragt, die jedoch eine hohe Augensicherheit aufweisen müssen, um gesundheitliche Schäden durch ungewolltes Einstrahlen in das menschliche Auge sicher ausschließen zu können.

In den genannten Anwendungsgebieten werden beispielsweise Blitzlampen oder Super-Lumineszenzdioden eingesetzt, die jedoch einen geringen Wirkungsgrad aufweisen. Zwar haben Laserdioden einen hohen Wirkungsgrad, jedoch eine für viele Anwendung zu geringe Augensicherheit. Zur Überwindung der genannten Nachteile ist es aus US 7,118,563 B2 bekannt, Laserdioden mit hohem Wirkungsgrad zur Erhöhung der Augensicherheit mit konkaven Linsen oder Diffusoren zu kombinieren. Zwar kann dadurch die Augensicherheit erhöht werden, jedoch wird der Wirkungsgrad nachteilhafterweise reduziert. Weiterhin nachteilig sind erhöhte Kosten und eine komplizierte Montage.

Aus EP1460741A1 ist ein Halbleiterbauelement mit den Merkmalen des Oberbegriffs des Anspruchs 1 bekannt.

Es ist daher Aufgabe der vorliegenden Erfindung, eine Lichtquelle mit gleichzeitig hohem Wirkungsgrad und hoher Augensicherheit anzugeben. Darüber hinaus soll die erfindungsgemäße Lichtquelle preiswert herstellbar und einfach montierbar sein. Diese Aufgaben werden erfindungsgemäß durch die Merkmale des Patentanspruchs 1 gelöst. Zweckmäßige Ausgestaltungen der Erfindung sind in den Unteransprüchen enthalten.

Die Idee der Erfindung besteht darin, die vertikale und/oder laterale Wellenführung in einer Laserdiode derart zu gestalten, dass entlang der vertikalen Richtung (fast axis) und/oder der lateralen Richtung (slow axis) die Fernfelddivergenz (95%-Winkel) mehr als 50°, bevorzugter mehr als 70° beträgt. Vorzugsweise wird die Laserdiode derart ausgebildet, dass die Fernfelddivergenz sowohl entlang der vertikalen Richtung als auch der lateralen Richtung mehr als 50°, bevorzugter mehr als 70° beträgt.

Dadurch kann in Abhängigkeit von der Laserleistung in einem gewissen Abstand Augensicherheit ohne zusätzlichen Aufwand (Linsen, Diffusoren) gewährleistet werden. Als Fernfelddivergenz wird derjenige vertikale und/oder laterale Winkel in einer Entfernung von der Austrittsfacette von größer als 1 mm verstanden, innerhalb dessen 95% der Strahlungsleistung eingeschlossen sind bzw. abgestrahlt werden.

Das erfindungsgemäße optoelektronische Halbleiterbauelement (Laserdiode) weist eine aktive Schicht auf, die zur Strahlungserzeugung geeignet ist, wobei eine erste Wellenleiterschicht auf einer ersten Seite der aktiven Schicht angeordnet, eine erste Mantelschicht auf der ersten Wellenleiterschicht angeordnet, eine zweite Wellenleiterschicht auf einer zweiten Seite der aktiven Schicht angeordnet, und eine zweite Mantelschicht auf der zweiten Wellenleiterschicht angeordnet sind, wobei sich die erste Seite und die zweite Seite in Bezug auf die aktive Schicht gegenüberliegen, wobei eine Reflexionsfacette zur Reflexion der von der aktiven Schicht emittierten Strahlung und eine Austrittsfacette zur partiellen Reflexion und partiellen Auskopplung der von der aktiven Schicht emittierten Strahlung vorgesehen sind, wobei die Reflexionsfacette und die Austrittsfacette jeweils im Randbereich der aktiven Schicht angeordnet sind und wobei sich die Reflexionsfacette und die Austrittsfacette in Bezug auf die aktive Schicht gegenüberliegen, wobei die aktive Schicht, die erste Mantelschicht, die erste Wellenleiterschicht, die zweite Wellenleiterschicht und die zweite Mantelschicht erfindungsgemäß zur Erhöhung der vertikalen Fernfelddivergenz derart ausgebildet sind, dass die Bedingungen 0,01 µm ≤ d_{wL} ≤ 1,0 µm und Δn ≥ 0,04 erfüllt sind, wobei d_{wL} die Summe der Schichtdicke der ersten Wellenleiterschicht, der Schichtdicke der aktiven Schicht und der Schichtdicke der zweiten Wellenleiterschicht ist, und Δn ein Maximum der Brechzahldifferenz zwischen der ersten Mantelschicht und der ersten Wellenleiterschicht und der Brechzahldifferenz zwischen der zweiten Wellenleiterschicht und der zweiten Mantelschicht ist.

Es wurde gefunden, dass die (vertikale) Fernfelddivergenz bei einem (vertikalen) Schichtaufbau größer als 70° und somit die emittierte Strahlung augensicher im Sinne der vorliegenden Erfindung ist, wenn die Summe der Schichtdicken der Wellenleiter und der aktiven Schicht geeignet gewählt wird und weiterhin der (maximale) Brechzahlunterschied zwischen Wellenleiterschichten und zugehörigen Mantelschichten einen gewissen Wert überschreiten. Herkömmliche Designs von Laserdioden zielen darauf ab, die (vertikale und laterale) Fernfelddivergenz möglichst gering zu halten, um die Laserstrahlung gut fokussieren zu können. Entgegen der üblichen Herangehensweise wurde gefunden, dass eine Laserdiode mit einer hohen (vertikalen) Fernfelddivergenz (größer als 50°, bevorzugter größer 70°) sowohl eine hohen Wirkungsgrad als auch eine hohe Augensicherheit bietet. Daher ist die erfindungsgemäße Laserdiode besonders für Anwendungen geeignet, bei denen einerseits ein hoher Wirkungsgrad und andererseits eine hohe Augensicherheit erforderlich sind, beispielsweise bei kosmetischen Behandlungen.

Vorzugsweise sind die erste Mantelschicht, die erste Wellenleiterschicht, die zweite Wellenleiterschicht und die zweite Mantelschicht derart ausgebildet sind, dass die Bedingung Δn > 0,1, noch bevorzugter Δn > 0,15, noch bevorzugter Δn > 0,20, noch bevorzugter Δn > 0,25 und noch bevorzugter Δn > 0,30 erfüllt ist. Durch einen höheren Brechzahlunterschied zwischen Wellenleiterschicht und Mantelschicht kann die (vertikale) Fernfelddivergenz erfindungsgemäß weiter erhöht werden. Vorzugsweise sind die Brechzahlen von erster Wellenleiterschicht und zweiter Wellenleiterschicht gleich. Vorzugsweise sind die Brechzahlen von erster Mantelschicht und zweiter Mantelschicht gleich. Die Brechzahl bezieht sich auf die Zentralwellenlänge der von der aktiven Schicht emittierten Strahlung. Die Zentralwellenlänge der von der aktiven Schicht emittierten Strahlung liegt vorzugsweise zwischen 380 nm und 10 µm, bevorzugter zwischen 380 und 2000 nm.

Vorzugsweise sind die erste Wellenleiterschicht, die aktive Schicht und die zweite Wellenleiterschicht derart ausgebildet sind, dass die Bedingung 0,01 µm ≤ d_{wL} ≤ 0,75 µm, bevorzugter 0,01 µm ≤ d_{wL} ≤ 0,5 µm und noch bevorzugter 0,01 µm ≤ d_{wL} ≤ 0,3 µm erfüllt ist. Die oben genannten Werte für d_{wL} sind vorzugsweise für einen (Haarentfernungs-)Laser geeignet, der bei 800nm emittiert.

Die Schichtdickengrenzen für Strukturen mit einer anderen Emissionswellenlänge skalieren jeweils mit der internen Wellenlänge des Materials. Es wurde gefunden, dass die (vertikale) Fernfelddivergenz und damit Augensicherheit insbesondere für die angegebenen Bereiche weiter erhöht werden kann.

Vorzugsweise erstreckt sich die aktive Schicht über den gesamten Bereich zwischen der Reflexionsfacette und der Austrittsfacette. Vorzugsweise kontaktiert die aktive Schicht sowohl die Reflexionsfacette als auch die Austrittsfacette direkt.

Zur weiteren Erhöhung der lateralen Fernfelddivergenz wird ein Rippenwellenleiter vorgesehen, wobei der Rippenwellenleiter mit einem effektiven Indexsprung Δn_{eff} > 0,06, bevorzugt mit einem effektiven Indexsprung Δn_{eff} > 0,10 ausgebildet ist. Der effektive Indexsprung ist die Differenz des effektiven Brechungsindexes im Bereich des Rippenwellenleiters und des effektiven Brechungsindexes im Bereich neben dem Rippenwellenleiter. Die sogenannte effektive Indexmethode zur Bestimmung des effektiven Brechungsindexes ist beispielsweise aus Coldren und Corzine, "Diode lasers and photonic integrated circuits" Wiley-Interscience, New York, 1995, S. 428-440, Appendix 3 ("Introduction to Optical Waveguide in Simple Double-Heterostructures") bekannt.

Vorzugsweise weist die erfindungsgemäße Laserdiode sowohl einen Rippenwellenleiter mit einem effektiven Indexsprung Δn_{eff} > 0,06, bevorzugt mit einem effektiven Indexsprung Δn_{eff} > 0,10 als auch Schichten (Mantelschichten, Wellenleiterschichten und aktive Schicht) mit den Bedingungen 0,01 µm ≤ d_{wL} ≤ 1,0 µm und Δn ≥ 0,04 auf (vorzugsweise bei einer Emissionswellenlänge des Lasers von 800nm - für Anwendungen mit λ ≠ 800nm wird d_{WL} vorzugsweise mit der internen Wellenlänge im Material skaliert). Ein entsprechender effektiver Indexsprung Δn_{eff} kann durch diverse Techniken realisiert werden, z.B. Ätzen und Überwachsen, Eindiffundierung, Implantation oder Rippenwellenleitertechniken.

Als bevorzugte Ausführungsvariante wird die Nutzung eines Rippenwellenleiters näher erläutert, jedoch ist die vorliegende Erfindung darauf nicht beschränkt.

Vorzugsweise ist der Rippenwellenleiter durch (zwei) Gräben ausgebildet, welche in die zweite Wellenleiterschicht und die zweite Mantelschicht (und ggf. darüber liegende Schichten) eingebracht (vorzugsweise geätzt) sind, wobei die Tiefe der Gräben derart ausgebildet ist, dass ein minimaler Abstand zwischen der der zweiten Wellenleiterschicht zugewandten Seite der aktiven Schicht und der der aktiven Schicht zugewandten Seite der Gräben größer als 100 nm ist. Mit anderen Worten wird der vom Substrat aus oben liegende Wellenleiter vorzugsweise möglichst tief geätzt, wobei nicht in die aktive Schicht durchgeätzt werden soll. Dadurch wird die Zuverlässigkeit des Bauelements erhöht. Besonders bevorzugt wird ein oben liegender, asymmetrischer (vorzugsweise p-) Wellenleiter verwendet, der im Vergleich zum anderen (n-)Wellenleiter eine große Dicke aufweist. Das hat einerseits den Vorteil, dass der oben liegende Wellenleiter sehr tief geätzt werden kann (sehr tiefe Gräben), ohne die aktive Schicht durchzuätzen, wodurch ein hoher effektiver Indexsprung, also eine hohe laterale Fernfelddivergenz erreicht werden kann. Andererseits kann durch die Asymmetrie eine hohe Zuverlässigkeit dadurch erreicht werden, weil nur ein geringer Teil der Strahlungsleistung in der aktiven Schicht liegt, wodurch ein COMD (catastrophal optical mirror damage) trotz hoher Leistung verhindert werden kann.

Vorzugsweise sind die erste Mantelschicht und die erste Wellenleiterschicht n-leitend und die zweite Wellenleiterschicht und die zweite Mantelschicht p-leitend ausgebildet. Vorzugsweise sind die erste Wellenleiterschicht, die aktive Schicht und die zweite Wellenleiterschicht derart ausgebildet, dass das Verhältnis zwischen der Schichtdicke der zweiten Wellenleiterschicht einerseits und der Summe der Schichtdicke der ersten Wellenleiterschicht, der Schichtdicke der aktiven Schicht und der Schichtdicke der zweiten Wellenleiterschicht andererseits größer als 0,45, bevorzugter größer 0,5, noch bevorzugter größer 0,55, noch bevorzugter größer 0,60, noch bevorzugter bevorzugt größer 0,65, noch bevorzugter größer 0,70, noch bevorzugter größer 0,75, noch bevorzugter größer 0,80, noch bevorzugter größer 0,85 und noch bevorzugter größer 0,9 ist. Es wurde gefunden, dass ein großes Verhältnis der (vorzugsweise p-) Wellenleiterdicke zur Gesamtdicke von aktiver Schicht und Wellenleitern zu einem großen effektiven Indexsprung Δn_{eff} und entsprechend zu einer großen lateralen Fernfelddivergenz führt.

Vorzugsweise werden die Gräben nah an die aktive Schicht herangeführt, ohne die aktive Schicht jedoch zu durchdringen. Vorzugsweise ist ein minimaler Abstand zwischen der der zweite Wellenleiterschicht zugewandten Seite der aktiven Schicht und der der aktiven Schicht zugewandten Seite der Gräben kleiner als 500 nm, bevorzugter kleiner als 250 nm.

Die richtige Auswahl der Breite des Rippenwellenleiters ist ein Gleichgewicht zwischen diverse Effekten. Erstens haben praktikabel herstellbare Rippenwellenleiter eine Breite, die aufgrund von Prozesstoleranzen im Bereich > 1µm liegt. Breite Rippenwellenleiter führen mehr als eine laterale Mode. Moden höherer Ordnung haben breitere laterale Winkel und sind für eine hohe Augensicherheit und große Beleuchtungsvolumen bevorzugt. Jedoch spielt nicht jede Obermode eine Rolle im Laserbetrieb, typisch nur die ersten paar Moden. Strukturen mit sehr großen Rippenwellenleiterbreiten >> 50µm erzeugen viele Obermoden, jedoch haben die ersten paar Moden, die eine entscheidende Rolle im Laserbetrieb spielen, relativ schmale Ausgangswinkel. Mit den vorgenannten vertikalen Schichtdicken erhält man eine bevorzugte laterale Fernfelddivergenz, d.h. die Laserdioden mit lateralen Ausgangswinkeln in Bereich > 70° (FF95%, Fernfelddivergenz, in der 95% der Strahlungsenergie eingeschlossen sind).

Bevorzugte Breiten des Rippenwellenleiters sind d_{well} = 1-20µm, noch bevorzugter d_{well} = 2-10µm und noch bevorzugter d_{well} = 3-7µm. Für Anwendungen mit λ ≠ 800nm ist es bevorzugt, die Breite mit der internen Wellenlänge im Material zu skalieren.

Vorzugsweise weist das Bauelement weder einen Diffusor noch eine konkave Linse auf. Vorzugsweise weisen alle Schichten (insbesondere aktive Schicht, Wellenleiterschichten und Mantelschichten) eine relativ uniforme Schichtdicke über ihre Gesamterstreckung auf, wobei das Verhältnis von maximaler Schichtdicke zu minimaler Schichtdicke kleiner als 2 ist. Vorzugsweise weisen alle Schichten (insbesondere aktive Schicht, Wellenleiterschichten und Mantelschichten) eine uniforme Schichtdicke auf.

### Kurzbeschreibung der Zeichnungen

Die Erfindung wird nachfolgend anhand von Ausführungsbeispielen näher erläutert. Es zeigen:
- Fig. 1A: die erfindungsgemäße Laserdiode in schematischer perspektivischer Darstellung,
- Fig. 1B: die erfindungsgemäße Laserdiode aus Fig. 1 A in schematischer geschnittener Darstellung entlang einer Achse quer zur Lichtausbreitungsrichtung,
- Fig. 1C: die erfindungsgemäße Laserdiode aus Fig. 1 A in schematischer geschnittener Darstellung entlang einer Achse parallel zur Lichtausbreitungsrichtung,
- Fig. 2: die Abhängigkeit der vertikalen Fernfelddivergenz der erfindungsgemäßen Laserdiode von der Gesamtdicke der Wellenleiterschichten inklusive aktiver Schicht einerseits und der Brechzahldifferenz zwischen Wellenleiterschichten und Mantelschichten andererseits,
- Fig. 3: die Brechzahlverteilung entlang der Schichten der erfindungsgemäßen Laserdiode mit symmetrischen Wellenleiterschichten sowie die dazugehörige vertikale Verteilung der Modenintensität in der erfindungsgemäßen Laserdiode,
- Fig. 4: die Brechzahlverteilung entlang der Schichten der erfindungsgemäßen Laserdiode mit asymmetrischen Wellenleiterschichten sowie die dazugehörige vertikalen Verteilung der Modenintensität in der erfindungsgemäßen Laserdiode,
- Fig. 5: die Abhängigkeit der in der aktiven Schicht anteiligen Strahlungsenergie in Bezug auf die Gesamtstrahlungsenergie vom Verhältnis der p-Wellenleiterschichtdicke zur der Gesamtdicke der Wellenleiterschichten inklusive aktiver Schicht einerseits und die Abhängigkeit des Verhältnisses von Schichtdicke der aktiven Schicht zur in der aktiven Schicht anteiligen Strahlungsenergie vom Verhältnis der p-Wellenleiterschichtdicke zur der Gesamtdicke der Wellenleiterschichten inklusive aktiver Schicht andererseits,
- Fig. 6: eine bevorzugte Ausführungsvariante einer erfindungsgemäßen Laserdiode mit Rippenwellenleiter in schematischer geschnittener Darstellung,
- Fig. 7: die Abhängigkeit des effektiven Indexsprungs Δn_{eff} vom Verhältnis der p-Wellenleiterschichtdicke zur Gesamtdicke der Wellenleiterschichten inklusive aktiver Schicht, und
- Fig. 8: die Abhängigkeit der vertikalen Fernfelddivergenz vom effektiven Indexsprung Δn_{eff} einerseits und von der Breite des Rippenwellenleiters andererseits.

### Ausführliche Beschreibung der Zeichnungen

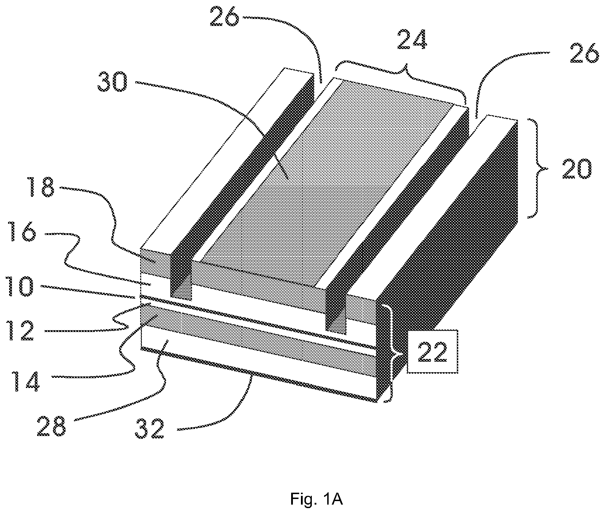

Fig. 1A-1C zeigen die erfindungsgemäße Laserdiode in perspektivischer und geschnittener Darstellung.

Die erfindungsgemäße Laserdiode weist einen vertikalen Schichtaufbau mit einem Substrat 28, einer darauf angeordneten, ersten n-leitenden Mantelschicht 14, einer darauf angeordneten, ersten n-leitenden Wellenleiterschicht 12, einer darauf angeordneten aktiven Schicht 10, einer darauf angeordneten, zweiten p-leitenden Wellenleiterschicht 16 und einer darauf angeordneten, zweiten p-leitenden Mantelschicht 18 auf.

Weiterhin weist die erfindungsgemäße Laserdiode an den seitlichen, gegenüberliegenden Enden eine Reflexionsfacette 20 mit einer hohen Reflektivität für die Zentralwellenlänge der von der aktiven Schicht 10 emittieren Strahlung und eine Austrittsfacette 22 mit einer Reflektivität, die eine Auskopplung der Strahlung ermöglicht, auf. Die Reflektivität der Reflexionsfacette 20 beträgt vorzugsweise größer 0,8, noch bevorzugter größer 0,9 und noch bevorzugter größer 0,99. Die Reflektivität der Austrittsfacette 22 ist kleiner als die Reflektivität der Reflexionsfacette 20.

Weiterhin weist die erfindungsgemäße Laserdiode Kontakte 30 und 32 zur Injektion von Ladungsträgern auf. Durch die so gebildete Struktur können Ladungsträger einer ersten Polarität jeweils über den ersten Kontakt 30, die erste Mantelschicht 14 (hier vorzugsweise n-leitend) und die erste Wellenleiterschicht 12 (hier vorzugsweise n-leitend) in die aktive Schicht 10 und Ladungsträger der entgegengesetzten Polarität jeweils über den zweiten Kontakt 32, die zweite Mantelschicht 18 (hier vorzugsweise p-leitend) und die zweite Wellenleiterschicht 16 (hier vorzugsweise p-leitend) ebenfalls in die aktive Schicht 10 gelangen und dort rekombinieren, wodurch eine Emission hervorgerufen wird. Die Facetten 20 und 22 bilden eine Kavität, so dass ein Laserbetrieb erreicht werden kann.

Die konkrete Struktur des in den Fig. 1A-1C gezeigten, bevorzugten Ausführungsbeispiels ist ein Diodenlaser mit einer aktiven Schicht 10 mit einer Emissionswellenlänge bei 808nm, hergestellt aus AlGaAs, mit einem GaAsP Einzelquantentrog. Mantel- und Wellenleiterschichten 12, 14, 16, 18 sind aus Al(x)Ga(1-x)As hergestellt. Für den Wellenleiter 12, 16 ist x in Al(x)Ga(1-x)As bevorzugt zwischen 85% und 20%, noch bevorzugter zwischen 70%-25% noch und bevorzugter zwischen 50%-30%.

Um das Δn-Ziel zu erfüllen, ist x für die Mantelschichten 14, 18 in Al(x)Ga(1-x)As bevorzugt > +5% verglichen mit der Aluminium-Gehalt der Wellenleiterschichten 12, 16, noch bevorzugter > +10%, noch bevorzugter > +25% und noch bevorzugter > +50% (jedoch maximal 100%). Die Dicke des Wellenleiters 24 ist bevorzugt 0,01-1,0 µm; noch bevorzugter 0,05-0,7µm und noch bevorzugter 0,1-0,5µm. Das Verhältnis der Dicke des oberen Wellenleiters 16 (hier p-dotiert) zur Dicke des unteren Wellenleiters 12 (hier n-dotiert) ist bevorzugt > 65%, bevorzugter > 75% und noch bevorzugter > 85%. Die Ätztiefe des Rippenwellenleiters 24 ist bevorzugt so gewählt, dass der geätzte Bereich 100nm von der aktiven Schicht 10 (entlang einer vertikalen Achse) entfernt ist. Die Breite des Rippenwellenleiters 24 ist bevorzugt 1-20µm, bevorzugter 2-10µm und noch bevorzugter 3-7µm.

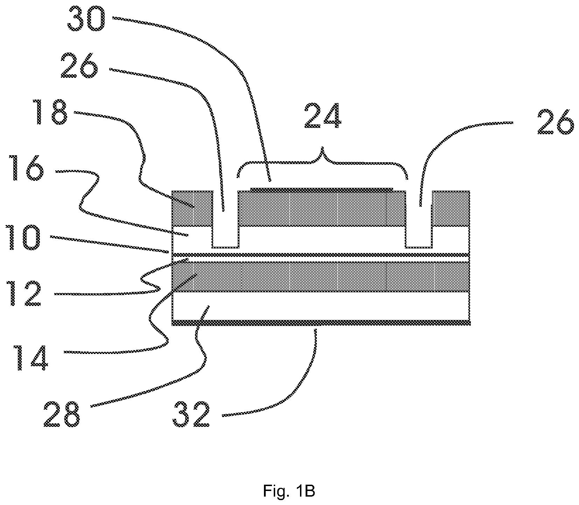

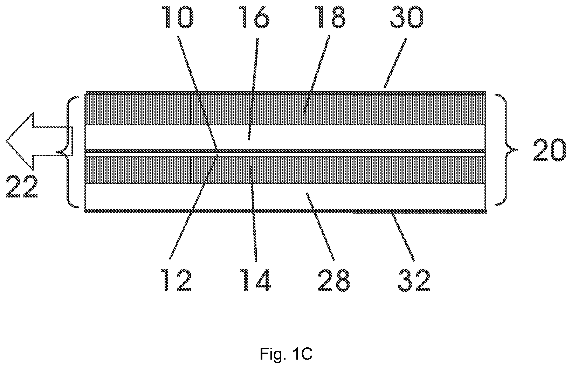

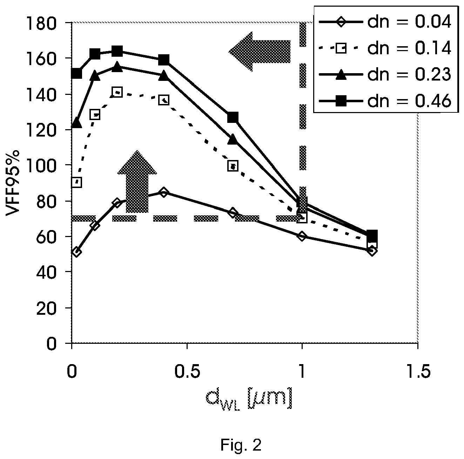

Fig. 2 zeigt die Abhängigkeit der vertikalen Fernfelddivergenz (VFF95%, vertikale Fernfelddivergenz, in der 95% der vertikalen Strahlungsenergie eingeschlossen sind) der Laserdiode der Fig. 1 von der Gesamtdicke d_{wL}, die sich aus der Summe der ersten Wellenleiterschicht 12, der aktiven Schicht 10 und der zweite Wellenleiterschicht 16 ergibt. Die Abhängigkeit ist für unterschiedliche Brechzahldifferenzen Δn zwischen erster Mantelschicht 14 und erster Wellenleiterschicht 12 dargestellt. Im vorliegenden Ausführungsbeispiel ist die Brechzahldifferenz Δn zwischen erster Mantelschicht 14 und erster Wellenleiterschicht 12 gleich Brechzahldifferenz zwischen zweiter Mantelschicht 18 und zweiter Wellenleiterschicht 16. Es ist zu erkennen, dass eine ausreichend hohe vertikale Fernfelddivergenz für eine Gesamtdicke d_{wL} zwischen 0,01 µm ≤ d_{wL} ≤ 1,0 µm und eine Brechzahldifferenz für Δn (bzw. Δn) ≥ 0,04 (bzw. Δn) erreicht werden kann. Besonders hohe vertikale Fernfelddivergenzen lassen sich für eine Gesamtdicke d_{wL} zwischen 0,01 µm ≤ d_{wL} ≤ 0,5 µm und eine Brechzahldifferenz für Δn (bzw. Δn) ≥ 0,2 erreichen.

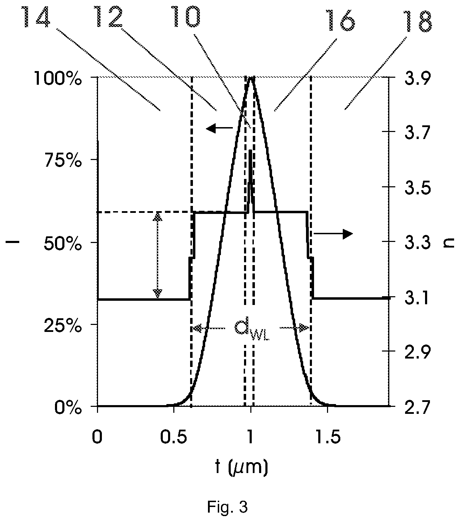

Fig. 3 zeigt die Brechzahlverteilung (Brechzahl n) entlang der Schichten 10, 12, 14, 16 und 18 entlang einer vertikalen Achse mit symmetrischen Wellenleiterschichten (Schichtdicke 12 = Schichtdicke 16) sowie die dazugehörige vertikale Verteilung der Modenintensität I. Die genaue vertikale Position (Tiefe) der einzelnen Schichten 10, 12, 14, 16 und 18 entlang der vertikalen Achse ist mit t bezeichnet. Weiterhin zeigt Fig. 3 zeigt die Brechzahldifferenz (Δn) zwischen den Schichten 12 und 14 sowie 16 und 18 und die Gesamtdicke d_{wL}.

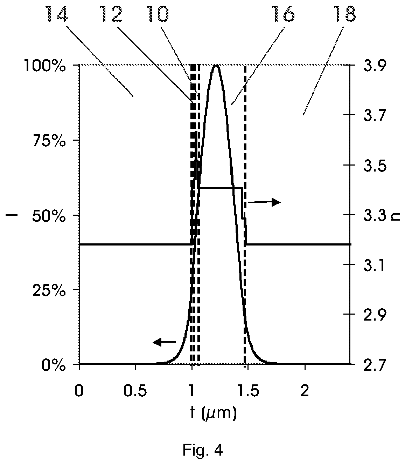

Fig. 4 zeigt wie Fig. 3 die Brechzahlverteilung sowie die dazugehörige vertikale Verteilung der Modenintensität I für asymmetrische Wellenleiterschichten, bei denen die Dicke der ersten Wellenleiterschicht 12 deutlich kleiner als die Dicke der zweiten Wellenleiterschicht 16 ist. Wie aus Fig. 4 ersichtlich ist, kann dadurch der Energieanteil in der aktiven Schicht 10 deutlich reduziert werden, wodurch vorteilhafterweise ein COMD trotz hoher Leistung der Laserdiode verhindert werden kann.

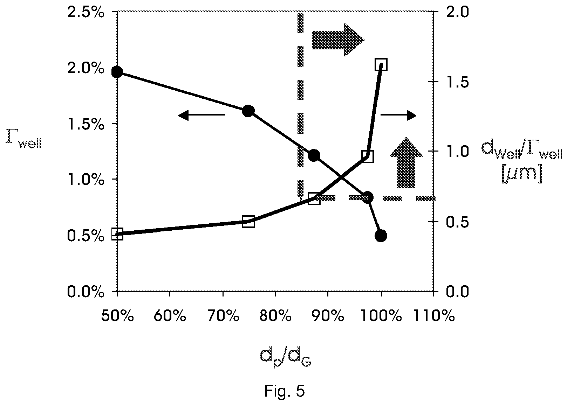

Fig. 5 zeigt einerseits die Abhängigkeit der in der aktiven Schicht 10 anteiligen Strahlungsenergie Γ_{WELL} in Bezug auf die Gesamtstrahlungsenergie vom Verhältnis der p-Wellenleiterschichtdicke d_{P} (=Schichtdicke der zweiten Wellenleiterschicht 16) zur der Gesamtdicke d_{G} der Wellenleiterschichten 12, 16 inklusive aktiver Schicht 10. Mit anderen Worten repräsentiert die anteilige Strahlungsenergie Γ_{WELL} das Verhältnis von Strahlungsenergie in der aktiven Schicht 10 zur Strahlungsenergie im gesamten Bauelement. Es ist aus Fig. 5 ersichtlich, dass die anteilige Strahlungsenergie Γ_{WELL} mit zunehmendem Verhältnis d_{P}/ d_{G} sinkt. Darüber hinaus zeigt Fig. 5 die Abhängigkeit des Verhältnisses von Schichtdicke d der aktiven Schicht 10 zur in der aktiven Schicht 10 anteiligen Strahlungsenergie Γ_{WELL} vom Verhältnis d_{P}/ d_{G} der p-Wellenleiterschichtdicke (Schichtdicke von 16) zur der Gesamtdicke der Wellenleiterschichten inklusive aktiver Schicht (Summe der Schichtdicken von 12, 10, 16). Es ist aus Fig. 5 ersichtlich, dass das Verhältnis d/ Γ_{WELL} mit zunehmendem Verhältnis d_{P}/ d_{G} steigt.

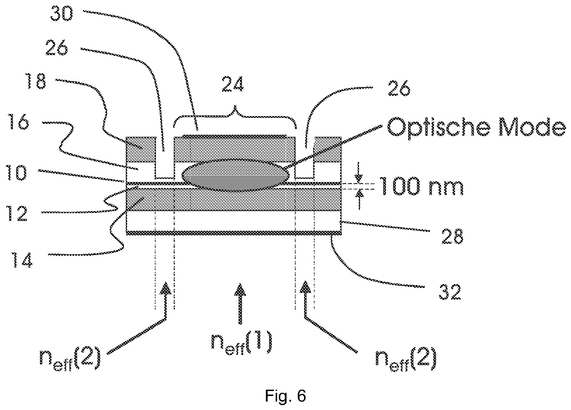

Fig. 6 zeigt eine bevorzugte Ausführungsvariante einer erfindungsgemäßen Laserdiode mit Rippenwellenleiter 24 in schematischer geschnittener Darstellung. Durch den Rippenwellenleiter 24 wird ein lateraler Indexsprung Δn_{eff} erzeugt. Zur Bestimmung des lateralen Indexsprungs Δn_{eff} kann die sogenannte effektive Indexmethode zur Bestimmung der effektiven Brechungsindices n_{eff}(1) und n_{eff}(2) verwendet werden. Zur Vergrößerung der lateralen Fernfelddivergenz sollen die Gräben 26 möglichst tief, jedoch zur Verbesserung der Stabilität des Bauelements nicht in die aktive Schicht 10 geätzt werden. Vorzugsweise bleibt ein Abstand zwischen den Gräben 26 und der aktiven Schicht 10 von mindestens 100 nm. Die Materialien der Schichten 16 und 18 sowie die Dimension des Rippenwellenleiters 24 (und der Gräben 28) werden so gewählt, dass ein ausreichend hoher lateraler Indexsprung Δn_{eff} und damit eine ausreichend hohe laterale Fernfelddivergenz erreicht wird.

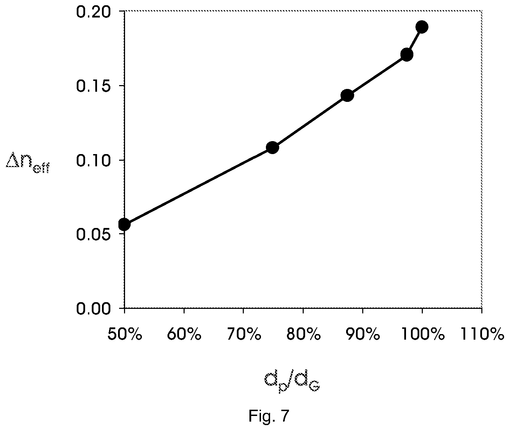

Fig. 7 zeigt die Abhängigkeit des effektiven Indexsprungs Δn_{eff} vom Verhältnis d_{P}/ d_{G} der p-Wellenleiterschichtdicke (Schichtdicke von 16) zur Gesamtdicke der Wellenleiterschichten inklusive aktiver Schicht (Summe der Schichtdicken von 12, 10, 16). Es ist ersichtlich, dass ein ausreichend hoher Indexsprung Δn_{eff} bei einem Verhältnis d_{P}/ d_{G} von größer 0,65 (bzw. 65%) erreicht werden kann.

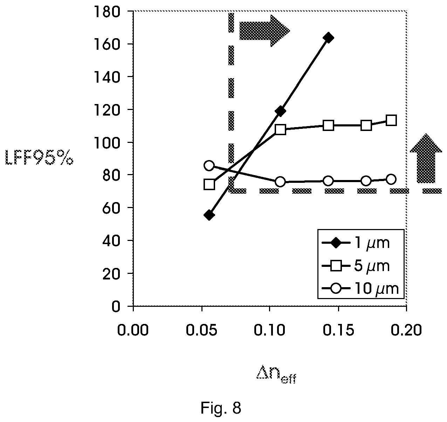

Fig. 8 zeigt die Abhängigkeit der lateralen Fernfelddivergenz (LFF95%, laterale Fernfelddivergenz, in der 95% der lateralen Strahlungsenergie eingeschlossen sind) vom effektiven Indexsprung Δn_{eff} einerseits und von der Breite des Rippenwellenleiters 24 andererseits. Es ist ersichtlich, dass eine ausreichend hohe laterale Fernfelddivergenz für Breiten des Rippenwellenleiters 24 von kleiner 10 µm, bevorzugt kleiner 5 µm und einem effektiven Indexsprung Δn_{eff} von größer 0,07 erreicht werden kann.

### Bezugszeichenliste

- 10: Aktive Schicht
- 12: Erste Wellenleiterschicht
- 14: Erste Mantelschicht
- 16: Zweite Wellenleiterschicht
- 18: Zweite Mantelschicht
- 20: Reflexionsfacette
- 22: Austrittsfacette
- 24: Rippenwellenleiter
- 26: Graben
- 28: Substrat
- 30: Kontakt zur Injektion von Ladungsträgern
- 32: Kontakt zur Injektion von Ladungsträgern

## Patentansprüche

1. Optoelektronisches Halbleiterbauelement, aufweisend:
eine aktive Schicht (10), die zur Strahlungserzeugung geeignet ist, eine erste Wellenleiterschicht (12), die auf einer ersten Seite der aktiven Schicht (10) angeordnet ist, eine erste Mantelschicht (14), die auf der ersten Wellenleiterschicht (12) angeordnet ist, eine zweite Wellenleiterschicht (16), die auf einer zweiten Seite der aktiven Schicht (10) angeordnet ist, und eine zweite Mantelschicht (18), die auf der zweiten Wellenleiterschicht (16) angeordnet ist, wobei sich die erste Seite und die zweite Seite in Bezug auf die aktive Schicht (10) gegenüberliegen, wobei eine Reflexionsfacette (20) zur Reflexion der von der aktiven Schicht (10) emittierten Strahlung und eine Austrittsfacette (22) zur Reflexion und Auskopplung der von der aktiven Schicht (10) emittierten Strahlung vorgesehen sind, wobei die Reflexionsfacette (20) und die Austrittsfacette (22) jeweils im Randbereich der aktiven Schicht (10) angeordnet sind und wobei sich die Reflexionsfacette (20) und die Austrittsfacette (22) in Bezug auf die aktive Schicht (10) gegenüberliegen, sodass die Bedingung (i) erfüllt ist:
(i) die aktive Schicht (10), die erste Mantelschicht (14), die erste Wellenleiterschicht (12), die zweite Wellenleiterschicht (16) und die zweite Mantelschicht (18) sind derart ausgebildet, dass die Bedingungen
0,01 µm ≤ d_{wL} ≤ 1,0 µm und Δn ≥ 0,04;
erfüllt sind,
wobei d_{wL} die Summe der Schichtdicke der ersten Wellenleiterschicht (12), der Schichtdicke der aktiven Schicht (10) und der Schichtdicke der zweiten Wellenleiterschicht (16) ist, und Δn ein Maximum der Brechzahldifferenz zwischen der ersten Wellenleiterschicht (12) und der ersten Mantelschicht (14) und der Brechzahldifferenz zwischen der zweiten Wellenleiterschicht (16) und der zweiten Mantelschicht (18) ist, **dadurch gekennzeichnet, dass** die Bedingung (ii) erfüllt ist:
(ii) das Halbleiterbauelement weist einen Rippenwellenleiter (24) mit einem effektiven Indexsprung Δn_{eff} > 0,06 auf.

2. Halbleiterbauelement nach Anspruch 1,
**dadurch gekennzeichnet, dass**
die erste Wellenleiterschicht (12), die aktive Schicht (10) und die zweite Wellenleiterschicht (16) derart ausgebildet sind, dass das Verhältnis zwischen der Schichtdicke der zweiten Wellenleiterschicht (16) einerseits und Summe der Schichtdicke der ersten Wellenleiterschicht (12), der Schichtdicke der aktiven Schicht (10) und der Schichtdicke der zweiten Wellenleiterschicht (16) andererseits größer als 0,65 ist.

3. Halbleiterbauelement gemäß der alternative (ii) des Anspruchs 1,
**dadurch gekennzeichnet, dass**
der Rippenwellenleiter (24) einen effektiven Indexsprung Δn_{eff} > 0,1 aufweist.

4. Halbleiterbauelement nach einem der vorhergehenden Ansprüche,
**dadurch gekennzeichnet, dass**
die erste Mantelschicht (14), die erste Wellenleiterschicht (12), die zweite Wellenleiterschicht (16) und die zweite Mantelschicht (18) derart ausgebildet sind, dass die Bedingung Δn > 0,15 erfüllt ist.

5. Halbleiterbauelement nach Anspruch 4,
**dadurch gekennzeichnet, dass**
die erste Mantelschicht (14), die erste Wellenleiterschicht (12), die zweite Wellenleiterschicht (16) und die zweite Mantelschicht (18) derart ausgebildet sind, dass die Bedingung Δn > 0,30 erfüllt ist.

6. Halbleiterbauelement nach einem der vorhergehenden Ansprüche,
**dadurch gekennzeichnet, dass**
die erste Wellenleiterschicht (12), die aktive Schicht (10) und die zweite Wellenleiterschicht (16) derart ausgebildet sind, dass die Bedingung 0,01 µm ≤ d_{wL} ≤ 0,75 µm erfüllt ist.

7. Halbleiterbauelement nach Anspruch 6,
**dadurch gekennzeichnet, dass**
die erste Wellenleiterschicht (12), die aktive Schicht (10) und die zweite Wellenleiterschicht (16) derart ausgebildet sind, dass die Bedingung 0,01 µm ≤ d_{wL} ≤ 0,5 µm erfüllt ist.

8. Halbleiterbauelement nach einem der vorhergehenden Ansprüche,
**dadurch gekennzeichnet, dass**
sich die aktive Schicht (10) über den gesamten Bereich zwischen der Reflexionsfacette (20) und der Austrittsfacette (22) erstreckt, wobei die aktive Schicht (10) die Reflexionsfacette (20) und die Austrittsfacette (22) direkt kontaktiert.

9. Halbleiterbauelement nach einem der vorhergehenden Ansprüche,
**dadurch gekennzeichnet, dass**
die erste Wellenleiterschicht (12), die aktive Schicht (10) und die zweite Wellenleiterschicht (16) derart ausgebildet sind, dass das Verhältnis zwischen der Schichtdicke der zweiten Wellenleiterschicht (16) einerseits und Summe der Schichtdicke der ersten Wellenleiterschicht (12), der Schichtdicke der aktiven Schicht (10) und der Schichtdicke der zweiten Wellenleiterschicht (16) andererseits größer als 0,85 ist.

10. Halbleiterbauelement nach einem der vorhergehenden Ansprüche,
**dadurch gekennzeichnet, dass**
der Rippenwellenleiter (24) durch Gräben (26), welche in die zweite Wellenleiterschicht (16) und die zweite Mantelschicht (18) eingebracht sind, ausgebildet ist, wobei die Tiefe Gräben (26) derart ausgebildet ist, dass ein minimaler Abstand zwischen der der zweite Wellenleiterschicht (16) zugewandten Seite der aktiven Schicht (10) und der der aktiven Schicht (10) zugewandten Seite der Gräben (26) größer als 100 nm ist.

11. Halbleiterbauelement nach Anspruch 10,
**dadurch gekennzeichnet, dass**
ein maximaler Abstand zwischen der der zweite Wellenleiterschicht (16) zugewandten Seite der aktiven Schicht (10) und der der aktiven Schicht (10) zugewandten Seite der Gräben (26) kleiner als 500 nm ist.

12. Halbleiterbauelement nach Anspruch 11,
**dadurch gekennzeichnet, dass**
ein maximaler Abstand zwischen der der zweite Wellenleiterschicht (16) zugewandten Seite der aktiven Schicht (10) und der der aktiven Schicht (10) zugewandten Seite der Gräben (26) kleiner als 250 nm ist.

13. Halbleiterbauelement nach einem der vorhergehenden Ansprüche,
**dadurch gekennzeichnet, dass**
die Breite des Rippenwellenleiters (24) kleiner als 20 µm, bevorzugt kleiner als 10 µm ist.

14. Halbleiterbauelement nach einem der vorhergehenden Ansprüche,
**dadurch gekennzeichnet, dass**
die erste Mantelschicht (14) und die erste Wellenleiterschicht (12) n-leitend ausgebildet und die zweite Wellenleiterschicht (16) und die zweite Mantelschicht (18) p-leitend ausgebildet sind.

15. Halbleiterbauelement nach einem der vorhergehenden Ansprüche,
**dadurch gekennzeichnet, dass**
das Bauelement weder einen Diffusor noch eine konkave Linse aufweist.

## Claims

1. An optoelectronic semiconductor component, comprising:
an active layer (10) that is suitable for generating radiation, a first waveguide layer (12) positioned on a first side of the active layer (10), a first cladding layer (14) positioned on the first waveguide layer (12), a second waveguide layer (16) positioned on a second side of the active layer (10), and a second cladding layer (18) positioned on the second waveguide layer (16), wherein the first side and the second side are opposite with respect to the active layer (10), wherein a reflection facet (20) for reflecting the radiation emitted by the active layer (10) and an emission facet (22) for reflection and feed-out of the radiation emitted by the active layer (10) are provided, wherein the reflection facet (20) and the emission facet (22) are each positioned in the marginal area of the active layer (10) and wherein the reflection facet (20) and the emission facet (22) are positioned opposite one another with respect to the active layer (10) such that the condition is met:
(i) the active layer (10), the first cladding layer (14), the first waveguide layer (12), the second waveguide layer (16) and the second cladding layer (18) are designed such that
the conditions
0.01 µm ≤ d_{wL} ≤ 1.0 µm and Δn ≥ 0.04;
are met,
wherein d_{wL} is the sum total of the layer thickness of the first waveguide layer (12), the layer thickness of the active layer (10) and the layer thickness of the second waveguide layer (16), and Δn is a maximum of the refractive index difference between the first waveguide layer (12) and the first cladding layer (14) and the refractive index difference between the second waveguide layer (16) and the second cladding layer (18), **characterized in that** the condition (ii) is met:
(ii) the semiconductor component comprises a ridge waveguide (24) with an effective index step Δn_{eff} > 0.06.

2. The semiconductor component according to Claim 1,
**characterized in that**
the first waveguide layer (12), the active layer (10) and the second waveguide layer (16) are designed such that the ratio between the layer thickness of the second waveguide layer (16) on the one hand and the sum total of the layer thickness of the first waveguide layer (12), the layer thickness of the active layer (10), and the layer thickness of the second waveguide layer (16) on the other hand is greater than 0.65.

3. The semiconductor component according to alternative (ii) of Claim 1,
**characterized in that**
the ridge waveguide (24) comprises an effective index step Δn_{eff} > 0.1.

4. The semiconductor component according to any one of the preceding claims,
**characterized in that**
the first cladding layer (14), the first waveguide layer (12), the second waveguide layer (16) and the second cladding layer (18) are designed such that the condition Δn > 0.15 is met.

5. The semiconductor component according to Claim 4,
**characterized in that**
the first cladding layer (14), the first waveguide layer (12), the second waveguide layer (16) and the second cladding layer (18) are designed such that the condition Δn > 0.30 is met.

6. The semiconductor component according to any one of the preceding claims,
**characterized in that**
the first waveguide layer (12), the active layer (10) and the second waveguide layer (16) are designed such that the condition 0.01 µm ≤ d_{w}L ≤ 0.75 µm is met.

7. The semiconductor component according to Claim 6,
**characterized in that**
the first waveguide layer (12), the active layer (10) and the second waveguide layer (16) are designed such that the condition 0.01 µm ≤ d_{w}L ≤ 0.5 µm is met.

8. The semiconductor component according to any one of the preceding claims,
**characterized in that**
the active layer (10) extends across the entire region between the reflection facet (20) and the emission facet (22), wherein the active layer (10) is in direct contact with the reflection facet (20) and the emission facet (22).

9. The semiconductor component according to any one of the preceding claims,
**characterized in that**
the first waveguide layer (12), the active layer (10) and the second waveguide layer (16) are designed such that the ratio between the layer thickness of the second waveguide layer (16) on the one hand and the sum total of the layer thickness of the first waveguide layer (12), the layer thickness of the active layer (10) and the layer thickness of the second waveguide layer (16) on the other hand is greater than 0.85.

10. The semiconductor component according to any one of the preceding claims,
**characterized in that**
the ribbed waveguide (24) is formed by grooves (26) that are introduced into the second waveguide layer (16) and the second cladding layer (18), wherein the depth of the grooves (26) is such that a minimum distance between the side of the active layer (10) facing the second waveguide layer (10) and the side of the grooves (26) facing the active layer (10) is greater than 100 nm.

11. The semiconductor component according to Claim 10,
**characterized in that**
a maximum distance between the side of the active layer (10) facing the second waveguide layer (16) and the side of the grooves (26) facing the active layer (10) is smaller than 500 nm.

12. The semiconductor component according to Claim 11,
**characterized in that**
a maximum distance between the side of the active layer (10) facing the second waveguide layer (16) and the side of the grooves (26) facing the active layer (10) is smaller than 250 nm.

13. The semiconductor component according to any one of the preceding claims,
**characterized in that**
the ridge waveguide (24) is less than 20 µm in width, preferably less than 10 µm.

14. The semiconductor component according to any one of the preceding claims,
**characterized in that**
the first cladding layer (14) and the first waveguide layer (12) are n-type and the second waveguide layer (16) and the second cladding layer (18) are p-type.

15. The semiconductor component according to any one of the preceding claims,
**characterized in that**
the component comprises neither a diffuser nor a concave lens.

## Revendications

1. Composant semi-conducteur opto-électronique, comprenant :
une couche active (10), apte à générer un rayonnement, une première couche guide d'ondes (12) disposée sur une première face de la couche active (10), une première couche de recouvrement (14) disposée sur la première couche guide d'ondes (12), une deuxième couche guide d'ondes (16) disposée sur une deuxième face de la couche active (10), et une deuxième couche de recouvrement (18) disposée sur la deuxième couche guide d'ondes (16), où la première face et la deuxième face sont opposées par rapport à la couche active (10), où une facette de réflexion (20) est prévue pour le réflexion du rayonnement émis par la couche active (10) et une facette de sortie (22) est prévue pour la réflexion et le découplage du rayonnement émis par la couche active (10), où la facette de réflexion (20) et la facette de sortie (22) sont disposées chacune dans la zone de bord de la couche active (10) et où la facette de réflexion (20) et la facette de sortie (22) sont opposées par rapport à la couche active (10), si bien que la condition (i) est satisfaite :
(i) la couche active (10), la première couche de recouvrement (14), la première couche guide d'ondes (12), la deuxième couche guide d'ondes (16) et la deuxième couche de recouvrement (18) sont formées de manière à satisfaire aux conditions
0,01 µm ≤ d_{wL} ≤ 1,0 µm et Δn ≥ 0,04 ;
où d_{WL} est la somme de l'épaisseur de la première couche guide d'ondes (12), de l'épaisseur de la couche active (10) et de l'épaisseur de la deuxième couche guide d'ondes (16), et Δn un maximum de la différence d'indice de réfraction entre la première couche guide d'ondes (12) et la première couche de recouvrement (14) et de la différence d'indice de réfraction entre la deuxième couche guide d'ondes (16) et la deuxième couche de recouvrement (18),
**caractérisé en ce que** la condition (ii) est satisfaite :
(ii) le composant semi-conducteur présente un guide d'ondes nervuré (24) avec un saut d'indice effectif Δn_{eff} > 0,06.

2. Composant semi-conducteur selon la revendication 1,
**caractérisé en ce que**
la première couche guide d'ondes (12), la couche active (10) et la deuxième couche guide d'ondes (16) sont réalisées de telle manière que le rapport entre l'épaisseur de la deuxième couche guide d'ondes (16), d'une part, et la somme de l'épaisseur de la première couche guide d'ondes (12), de l'épaisseur de la couche active (10) et de l'épaisseur de la deuxième couche guide d'ondes (16), d'autre part, est supérieur à 0,65.

3. Composant semi-conducteur selon l'alternative (ii) de la revendication 1,
**caractérisé en ce que**
le guide d'ondes nervuré (24) présente un saut d'indice effectif Δn_{eff} > 0,1.

4. Composant semi-conducteur selon l'une des revendications précédentes,
**caractérisé en ce que**
la première couche de recouvrement (14), la première couche guide d'ondes (12), la deuxième couche guide d'ondes (16) et la deuxième couche de recouvrement (18) sont réalisées de manière à satisfaire la condition Δn > 0,15.

5. Composant semi-conducteur selon la revendication 4,
**caractérisé en ce que**
la première couche de recouvrement (14), la première couche guide d'ondes (12), la deuxième couche guide d'ondes (16) et la deuxième couche de recouvrement (18) sont réalisées de manière à satisfaire la condition Δn > 0,30.

6. Composant semi-conducteur selon l'une des revendications précédentes,
**caractérisé en ce que**
la première couche guide d'ondes (12), la couche active (10) et la deuxième couche guide d'ondes (16) sont réalisées de manière à satisfaire la condition 0,01 µm ≤ d_{wL} ≤ 0,75 µm.

7. Composant semi-conducteur selon la revendication 6,
**caractérisé en ce que**
la première couche guide d'ondes (12), la couche active (10) et la deuxième couche guide d'ondes (16) sont réalisées de manière à satisfaire la condition 0,01 µm ≤ d_{WL} ≤ 0,5 µm.

8. Composant semi-conducteur selon l'une des revendications précédentes,
**caractérisé en ce que**
la couche active (10) s'étend sur toute la zone entre la facette de réflexion (20) et la facette de sortie (22), la couche active (10) contactant directement la facette de réflexion (20) et la facette de sortie (22).

9. Composant semi-conducteur selon l'une des revendications précédentes,
**caractérisé en ce que**
la première couche guide d'ondes (12), la couche active (10) et la deuxième couche guide d'ondes (16) sont réalisées de telle manière que le rapport entre l'épaisseur de la deuxième couche guide d'ondes (16), d'une part, et la somme de l'épaisseur de la première couche guide d'ondes (12), de l'épaisseur de la couche active (10) et de l'épaisseur de la deuxième couche guide d'ondes (16), d'autre part, est supérieur à 0,85.

10. Composant semi-conducteur selon l'une des revendications précédentes,
**caractérisé en ce que**
le guide d'ondes nervuré (24) est formé par des sillons (26) creusés dans la deuxième couche guide d'ondes (16) et la deuxième couche de recouvrement (18), la profondeur des sillons (26) étant prévue de sorte qu'un espacement minimal entre la face de couche active (10) opposée à la deuxième couche guide d'ondes (16) et la face des sillons (26) opposée à la couche active (10) est supérieur à 100 nm.

11. Composant semi-conducteur selon la revendication 10,
**caractérisé en ce**
**qu'**un espacement maximal entre la face de couche active (10) opposée à la deuxième couche guide d'ondes (16) et la face des sillons (26) opposée à la couche active (10) est inférieur à 500 nm.

12. Composant semi-conducteur selon la revendication 11,
**caractérisé en ce qu'**un espacement maximal entre la face de couche active (10) opposée à la deuxième couche guide d'ondes (16) et la face des sillons (26) opposée à la couche active (10) est inférieur à 250 nm.

13. Composant semi-conducteur selon l'une des revendications précédentes,
**caractérisé en ce que**
la largeur du guide d'ondes nervuré (24) est inférieure à 20 µm, préférentiellement inférieure à 10 µm.

14. Composant semi-conducteur selon l'une des revendications précédentes,
**caractérisé en ce que**
la première couche de recouvrement (14) et la première couche guide d'ondes (12) sont conductrices N, et la deuxième couche guide d'ondes (16) et la deuxième couche de recouvrement (18) sont conductrices P.

15. Composant semi-conducteur selon l'une des revendications précédentes,
**caractérisé en ce que**
le composant ne comprend aucun diffuseur, ni aucune lentille concave.
